# EUROPEAN PATENT APPLICATION

(11) **EP 1 880 802 A2**
(43) Date of publication of application: **23.01.2008**
(21) Application number: 07251074.6
(22) Date of filing: 14.03.2007
(51) Int. Cl.: B25B 23/142

(54) **Semi automatic disposable torque limiting device and method**

(30) Priority: 20.07.2006 US 831953 P
(71) Applicant: Teleflex Medical Incorporated, Limerick, PA 19468-1699 (US)
(72) Inventor: Rinner, James A., Frnaksville, WI 53126 (US)
(74) Representative: Hedges, Martin Nicholas

(57) **Abstract**

In an apparatus that includes a head (20), spindle (28), and a handle (16), the handle is rotatably fastened to the head by the spindle. The handle and head mate at an interface. The apparatus further includes a head axial bore (26A) offset from the spindle and configured to occupy a portion of the head and a handle axial bore (26B) offset from the spindle and configured to occupy a portion of the handle, the handle axial bore being configured to receive a torque limiting insert (40), wherein in response to the head being at a first rotational conformation relative to the handle, the head axial bore and handle axial bore is disposed in cooperative alignment to allow the torque limiting insert to transect the interface and enter the head axial bore. The head and the handle are rotationally coupled via the torque limiting insert. The head and the handle are rotationally uncoupled in response to a shear force being applied across the interface that exceeds a shear limit for the torque limiting insert.

## Description

### CROSS REFERENCE TO RELATED APPLICATIONS

This application claims priority to U.S. Provisional Application Serial No. 60/831,953, filed on July 20, 2006, titled "SEMI AUTOMATIC DISPOSABLE TORQUE LIMITING DEVICE AND METHOD," the disclosure of which is incorporated herein by reference in its entirety.

The present invention generally relates to a torque limiting device and method. More particularly, the present invention pertains to a device and method for limiting an amount of torque applied by the device using a disposable insert.

In various manufacturing, construction, and medical industries, objects or fasteners are utilized that are positioned, threaded or screwed into place. These objects or fasteners may include a predetermined amount of torque that has been determined to be optimal for a given fastening or positioning situation. In addition, the fastener or object may include a predetermined amount of torque that has been determined to fatigue or break the fastener or object. Often, these predetermined torque values are determined by the manufacturer or by a testing facility. In use, a technician or user may employ a device such as a torque wrench to set the fastener or object according to the predetermined amount of torque. In a particular example, bone screws may be employed by surgeons to reconstruct bones or attach replacement components to bones of patients. In such circumstances, applying a proper amount of torque may be critically important.

Conventional torque wrenches utilize forces from coil springs and spring washers along with friction to limit the amount of torque applied. Unfortunately, as components within these conventional torque wrenches slide by one another, wear may alter the torque setting of the conventional torque wrench. As such, conventional torque wrenches need to be recalibrated to maintain their torque limit range.

Conventional torque wrenches require lubricants for the proper operation of the mechanism. These lubricants break down during steam-sterilization and can oxidize components which may reduce the accuracy of the device. In addition, these conventional torque wrenches require re-lubrication during cleaning or re-calibration cycles.

Conventional torque wrench mechanisms may also fail and bind or lock-up thereby eliminating the torque-limiting effect and essentially converting the tool to a rigid, nonlimiting tool with torque limiting now regulated to the tool users ability to discern torque forces by hand. This could lead to over-torquing, an unsafe condition.

Accordingly, it is desirable to provide a device and method capable of overcoming the disadvantages described herein at least to some extent.

The foregoing needs are met, to a great extent, by the present invention, wherein in one respect a device and method of limiting an amount of torque applied is provided.

An embodiment of the present invention pertains to a torque limiting apparatus. The torque limiting apparatus includes a head, handle, head axial bore, and handle axial bore. The head includes a central rotational axis. The handle is rotatably fastened to the head. The handle and head mate at an interface. The head axial bore is defined by the head and is offset from the central rotational axis. The handle axial bore is defined by the handle and offset from the central rotational axis. The handle axial bore is shaped to receive a torque limiting insert. In response to the head being at a first rotational position relative to the handle, the head axial bore and handle axial bore are disposed in cooperative alignment to allow the torque limiting insert to transect the interface and enter the head axial bore. The head and the handle are rotationally coupled via the torque limiting insert. The head and the handle are rotationally uncoupled in response to a shear force being applied across the interface that exceeds a shear limit for the torque limiting insert.

Another embodiment of the present invention relates to an apparatus for applying a predetermined amount of torque to a fastener. The apparatus includes a head means, handle means, and torque limiting means. The head means transmits torque to the fastener. The handle means is coupled to the head means for applying an amount of torque to the fastener. The torque limiting means is interoperably disposed between the head means and the handle means to limit an applied torque from being transmitted from the handle means to the head means by shearing a torque limiting insert in response to the amount of torque being greater than the predetermined amount of torque. The handle means is rotationally decoupled from the fastener in response to the torque limiting insert being sheared.

Yet another embodiment of the present invention pertains to a method of applying a predetermined amount of torque upon a fastener. In this method, a torque limiting device is coupled to the fastener. The torque limiting device includes a torque limiting insert. In addition, an amount of torque is applied upon the fastener by urging a handle of the torque limiting device to rotate. The torque limiting insert is subjected to a shear force in response to the amount of torque. The torque limiting insert is sheared in response to the amount of torque being greater than the predetermined amount of torque. The handle is rotationally decoupled from the fastener in response to the torque limiting insert being sheared.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.
FIG. 1 is a perspective view of the torque limiting device according to an embodiment of the invention.
FIG. 2 is an exploded view of the torque limiting device of FIG. 1.
FIG. 3A is a side view of a disposable insert suitable for use with the torque limiting device of FIG. 1.
FIG. 3B is a side view of the disposable insert following a calibration procedure.
FIG. 3C is an exploded view illustrating insertion of the disposable insert into the torque limiting device of FIG. 1.
FIG. 4A is a top view of the torque limiting device in a loaded position.
FIG. 4B is a cross-sectional view A-A of the torque limiting device in the loaded position.
FIG. 5A is a top view of the torque limiting device in a shear position.
FIG. 5B is a cross-sectional view B-B of the torque limiting device in the shear position.
FIG. 5C is an end view of the torque limiting device in the shear position.
FIG. 5D is a cross-sectional view C-C of the torque limiting device in the shear position.
FIG. 6A is a top view of the torque limiting device in an eject position.
FIG. 6B is a cross-sectional view D-D of the torque limiting device in the eject position.
FIG. 6C is an end view of the torque limiting device in the eject position.
FIG. 6D is a cross-sectional view E-E of the torque limiting device in the eject position.
FIG. 7A is a top view of the torque limiting device in a second loaded position.
FIG. 7B is a cross-sectional view F-F of the torque limiting device in the second loaded position.
FIG. 8A is a top view of the torque limiting device in a second shear position.
FIG. 8B is a cross-sectional view G-G of the torque limiting device in the second shear position.
FIG. 8C is an end view of the torque limiting device in the second shear position.
FIG. 8D is a cross-sectional view H-H of the torque limiting device in the second shear position.
FIG. 9A is a top view of the torque limiting device in a second eject position.
FIG. 9B is a cross-sectional view I-I of the torque limiting device in the second eject position.
FIG. 9C is an end view of the torque limiting device in the second eject position.
FIG. 9D is a cross-sectional view J-J of the torque limiting device in the second eject position.
FIG. 10A is a cross-sectional view of the torque limiting device in a final eject position.
FIG. 10B is a cross-sectional view of the torque limiting device in the final eject position.
FIG. 10C is a cross-sectional view of the torque limiting device in the final eject position according to another embodiment.
FIG. 11 is an exploded view illustrating removal of the used insert from the torque limiting device of FIG. 1.
FIG. 12 is a flow diagram illustrating a method of installing a fastener according to an embodiment of the invention.

The present invention provides a semi automatic torque limiting device and method. In one example of a preferred embodiment, the torque limiting device includes a disposable, torque limiting, insert. The disposable insert includes a predetermined number of segments. During use, a segment of the insert is sheared off in response to a predetermined amount of torque being applied to a handle of the torque limiting device. The sheared segment may be stored in the handle.

In various embodiments, the torque limiting device may include any suitable material, such as, plastic, polymer, resin, metal, or the like. In a preferred example, the torque limiting device includes materials that may be sterilized for use in an operating theater. For example, autoclaveable polymers and corrosion resistant-type metals may be utilized to fabricated the torque limiting device and/or the insert. The insert may include a rod or bar with a number of annular grooves that correspond to the number of segments. Material characteristics of the insert and the cross sectional area at the groove may be utilized to determine a torque value for the torque limiting device.

It is an advantage of embodiments of the invention that the torque limiting device substantially and/or continually maintains a pre-set torque limit that is governed by the insert. It is another advantage that this pre-set torque limit is essentially unaffected by frictional wear. As such, the need for testing and recalibration of the torque limiting device may be eliminated.

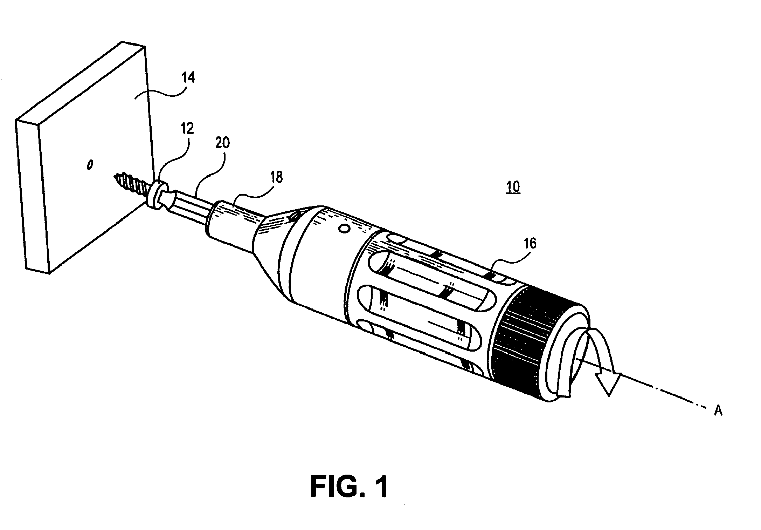

Preferred embodiments of the invention will now be described with reference to the drawing figures, in which like reference numerals refer to like parts throughout. As shown in FIG. 1, a torque limiting device 10 may be utilized to install a fastener 12 in a substrate 14. The torque limiting device 10 includes a handle 16 for the user, technician, or surgeon to grasp. The torque limiting device 10 further includes a shaft 18 connecting the handle 16 to a head 20. The head 20 is configured to engage the fastener 12. In various embodiments, the head 20 and shaft 18 may be essentially a single component or the head 20 may be releasably attached to the shaft 18 to facilitate exchanging the head 20 with another head 20.

In general, the torque limiting device 10 is configured such that in response to turning the handle 16 about an axis A (as shown in FIG. 1), an applied torque will urge the shaft 18 to rotate in a similar fashion. The torque limiting device 10 is configured such that at a threshold or max torque, the shaft 18 ceases to rotate about the axis A in response to rotating the handle 16 about the axis A. In conventional devices, the limit at this threshold torque is affected by frictional forces of one member sliding against another. Unfortunately, this friction results in mechanical wear that may alter the characteristics of the torque applied through the device. It is an advantage of various embodiments that friction is a substantially negligible component of the applied torque. As such, the embodiments are essentially unaffected by frictional or mechanical wear.

In addition, while not explicitly shown in FIG. 1, the torque limiting device 10 may include a display to display the amount of torque being applied to the fastener 12. Furthermore, the torque limiting device 10 may include a ratcheting mechanism to provide unidirectional or selectable unidirectional rotation of the shaft 18. This is an optional feature so that the operator does not have to change or reset the hand position on the tool.

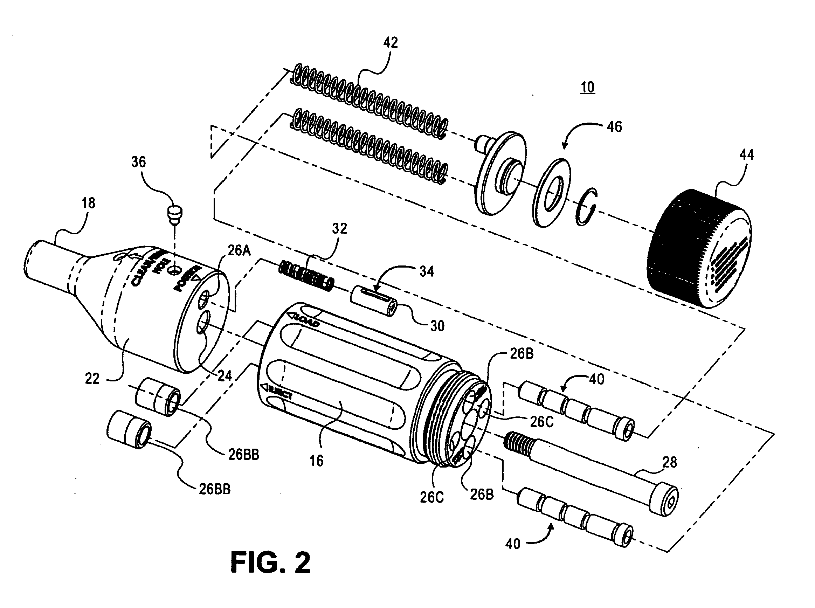

FIG. 2 is an exploded view of the torque limiting device of FIG. 1. On a driver-end of the device 10 there is the shear component 22. On the proximal end of the shear component 22 there are two holes: a center hole 24 and a shearing hole 26A. The center hole 24 receives a shoulder screw 28 that connects the rotating handle 16 to the driver-end 18. The shearing hole 26A carries a spring-loaded ejector pin 30 and a spring 32 whose travel is limited by the mating of an axial slot 34 in the ejector pin 30 and a limit pin 36.

The handle 16 includes a plurality of holes or bores that may be selectively aligned with the shearing hole 26A. For example, as shown in FIG. 2, the handle 16 includes an insert loading bore 26B and a segment collecting bore 26C. The insert loading bore 26B may further include a bushing 26BB to facilitate shearing the torque limiting insert 40.

In addition, the torque limiting device 10 includes one or more torque limiting disposable inserts or inserts 40, springs 42 to advance the inserts 40 and a cap 44 to retain the inserts 40 and a spring assembly 46.

FIG. 3A is a side view of the disposable insert 40 suitable for use with the torque limiting device 10. The inserts may be made from any suitable material. Suitable materials include plastic or polymers, metals, and the like. In a particular example, the inserts include a segmented plastic length of material. As shown in FIG. 3A, the insert 40 includes segments 50A to 50n that are delineated by annular grooves 52A to 52n. The annular grooves 52A to 52n serve to concentrate shear stress between adjacent segments 50A to 50n. In this manner, the annular grooves 52A to 52n facilitate a clean and/or repeatable shearing action. Because the shearing hole 26A is rotationally offset from the central axis A, torque applied to the handle 16 generates a corresponding force at the shearing hole 26A and a shearing force upon the torque limiting insert 40. In particular, the depth of the shearing hole 26A and length of the segments 50A to 50n are matched such that the shearing force is exerted, each in its turn, upon the annular grooves 52A to 52n. The cross sectional area and material characteristics of the torque limiting insert 40 at the annular grooves 52A to 52n is configure to shear in response to a predetermined amount of shear force. The amount of shear force each of the annular grooves 52A to 52n can absorb before being sheared or severed is a "shear limit." The shear limit is defined as the amount of shear stress or strain sufficient to result in the partial or complete fracture or breaking of the torque limiting insert 40. One or more of the segments 52A-52n may be sheared in the torque limiting device 10 or reasonable facsimile thereof to test or calibrate the insert 40. The insert 40 further includes a base or flanged insert portion such as a flange 54 shown in FIG. 3A.

In various embodiments, the shear limit of the torque limiting insert 40 may include any suitable value. For example, various standard fasteners may have a manufacturer's recommended installation torque of less than 1 Newton Meter (Nm) to greater than 10 Nm. Accordingly, the torque limiting insert 40 may include a shear limit of 1 Nm, 2 Nm, 4 Nm, 6 Nm, and the like. To differentiate the torque limiting insert 40 having one shear limit from another torque limiting insert 40 having another shear limit, the torque limiting insert 40 may include an indicator value, differentiating color, size, shape, or the like. In a particular example, the torque limiting insert 40 may include a particular shape for each shear limit. That is, while the torque limiting insert 40 shown in FIG. 3A is depicted as generally cylindrical, the torque limiting insert 40 having a different shear limit may be, for example, an elongated 3, 4, or multi-sided prism. According to an embodiment, the insert loading hole 26B and/or bushing 26BB may be configured to accept only a certain shaped torque limiting insert 40.

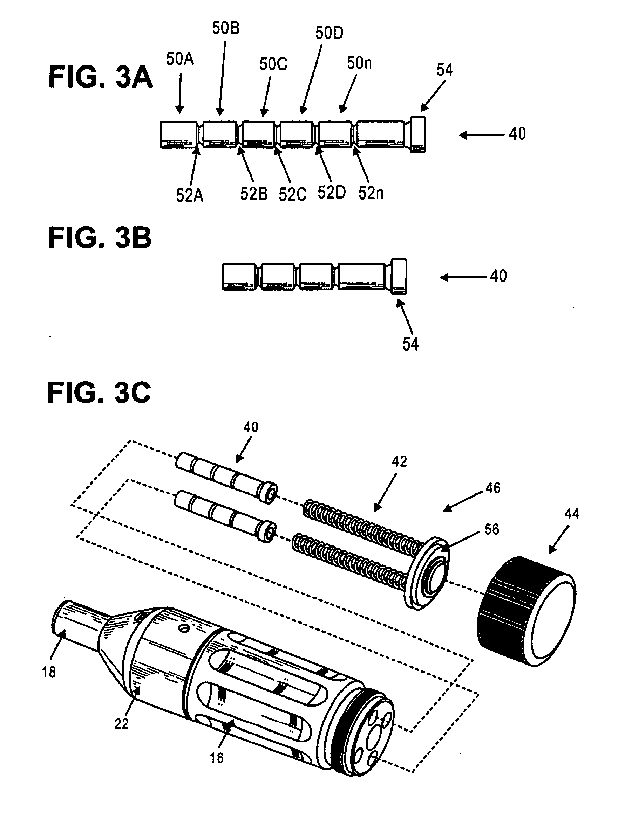

FIG. 3B is a side view of the disposable insert following a calibration procedure. Once the testing or calibration is performed a "working" length segmented insert 40 is left with three torque-limiting segments 50C to 50n remaining. However, in other embodiments, more or less than three segments 50A-50n may be retained.

FIG. 3C is an exploded view illustrating insertion of the disposable insert 40 into the torque limiting device 10. Loading the torque limiting device 10 is simple and a thrust bearing 56, for example, may be included on the backside of the spring assembly 46 to reduce assembly friction.

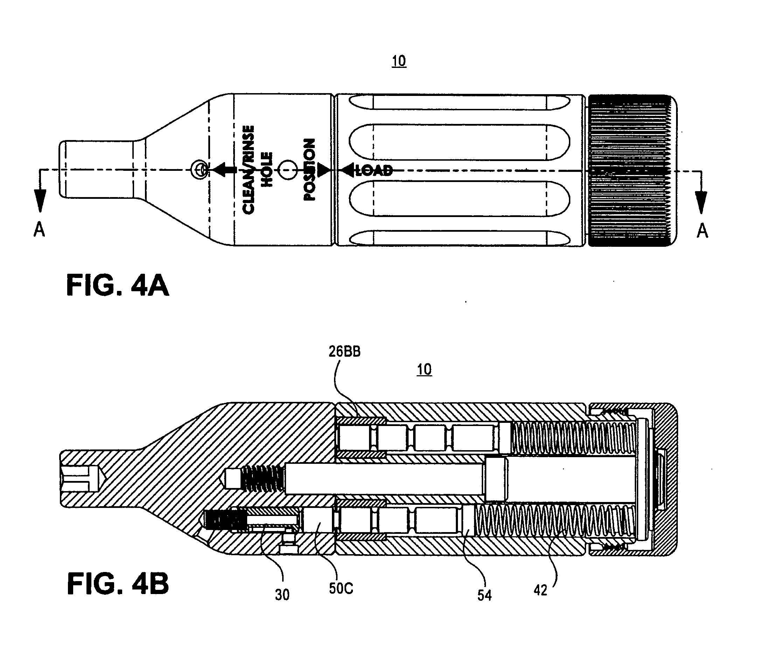

FIG. 4A is a top view of the torque limiting device 10 in a loaded position. The handle 16 is rotated to a "Load" position. At this point an insert 40 will be aligned with the eject pin 30.

FIG. 4B is a cross-sectional view A-A of the torque limiting device 10 in the loaded position. In the transparent view above, the loaded positions are shown. The handle 16 is turned till one of the insert 40, hereafter referred to as the "first insert 40," is loaded into the hole 26 in the shear component 22. There are springs 42 and 32 behind the first insert 40 and the ejector pin 30. The larger spring 42 behind the first insert 40 has a higher spring rate than the ejector pin spring 32, thus over-powering the ejector spring 32 and the first insert 40 advances the ejector pin 30 until its movement is limited by the pin-slot combination. When the first insert 40 is fully advanced to the stop the insert 40 is correctly positioned for the "torque-limiting" step.

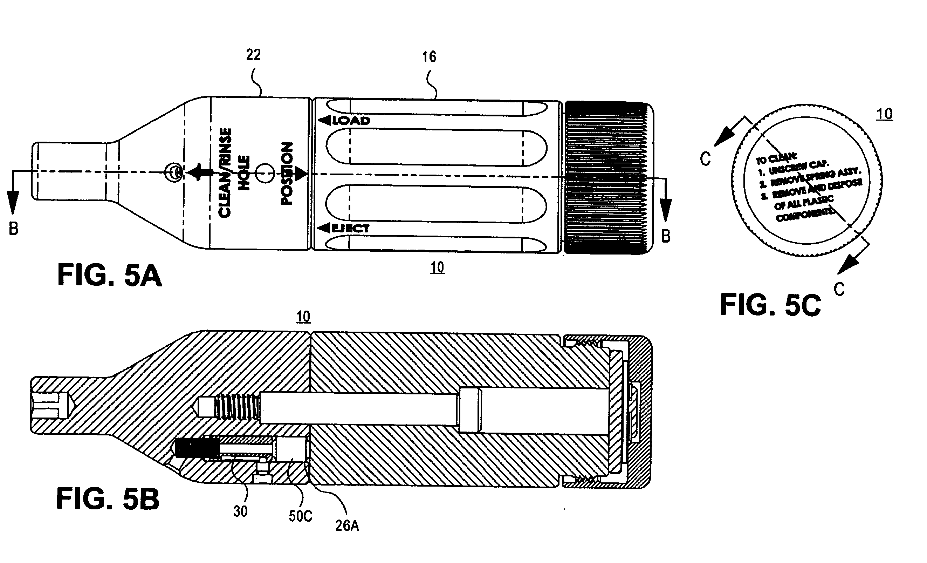

FIG. 5A is a top view of the torque limiting device 10 in a shear position. When the first insert 40 shears at the pre-determined torque value, the handle 16 will rotate relative to the shear component 22 to an in-between position between "Load" and "Eject".

FIG. 5B is a cross-sectional view B-B of the torque limiting device 10 in the shear position. In the above view the front segment 50C of the first insert 40 is shown sheared and retained inside the shearing hole 26A of the shear component 22. Because there is only the one shearing hole 26A on the shear component 22 that is on the same bolt circle as the first insert 40, the first insert 40 will not advance to the next position until the shearing hole 26A is emptied.

FIG. 5C is an end view of the torque limiting device in the shear position. As shown in FIG. 5C, the section line C-C is disposed at an angle to pass through the loading bores 26B.

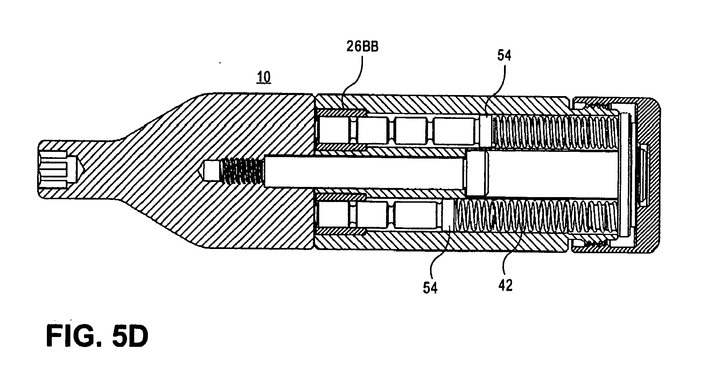

FIG. 5D is a cross-sectional view C-C of the torque limiting device 10 in the shear position. As shown in FIG. 5D, the cross-sectional view C-C transects the loading bore 26B and illustrates how both the first insert 40 and a second insert 40 are essentially prevented from advancing while the shear component 22 is positioned between "Load" and "Eject" position.

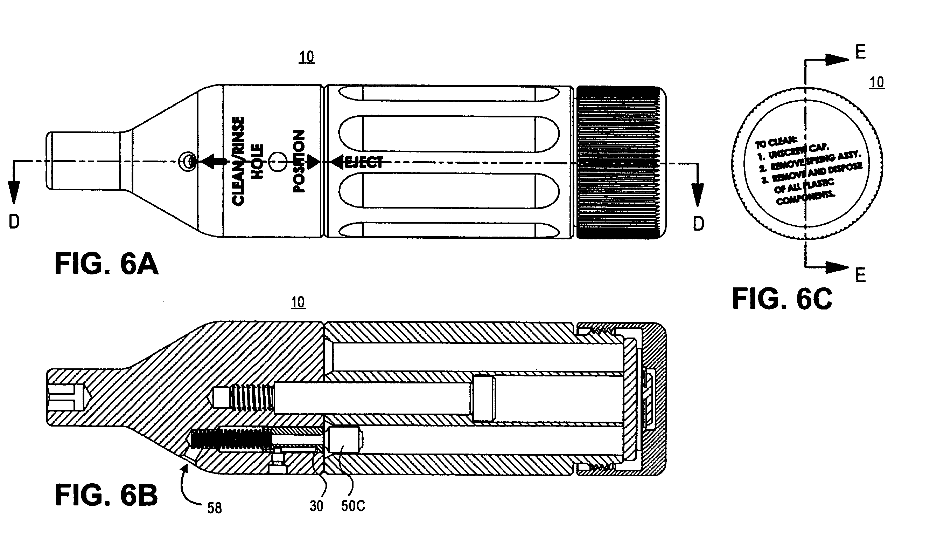

FIG. 6A is a top view of the torque limiting device 10 in an eject position. As shown in FIG. 6A, the handle 16 is rotated into an "Eject" position.

FIG. 6B is a cross-sectional view C-C of the torque limiting device 10 in the eject position. As shown in the cross-sectional view, the segment 50C of the first insert 40 that has been sheared off will now advance into ejection chamber of the handle 16. Because of the limit slot in the ejector pin 30 it will not advance into the ejection chamber. A cleaning port or clearing port 58 is optionally included to augment ejection. For example, if included, a wire or pin may be inserted to augment the action of the spring 32.

FIG. 6C is an end view of the torque limiting device 10 in the eject position. As shown in FIG. 6C, the section line E-E is disposed at an angle to pass through the loading bores 26B.

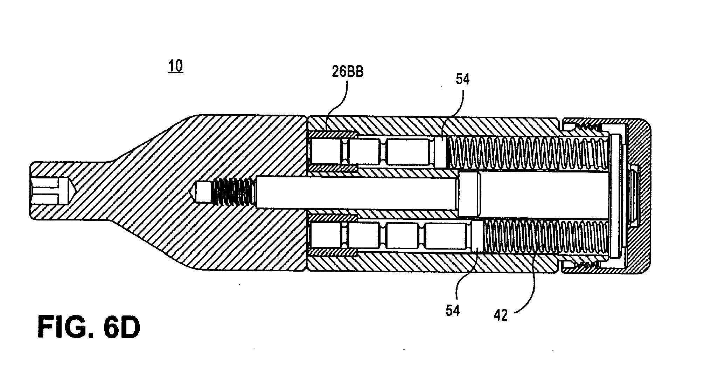

FIG. 6D is a cross-sectional view E-E of the torque limiting device 10 in the eject position. As shown in FIG. 6D, the cross-sectional view E-E transects the loading bore 26B and illustrates how both the first insert 40 and second insert 40 are essentially prevented from advancing while the shear component 22 is in the eject position.

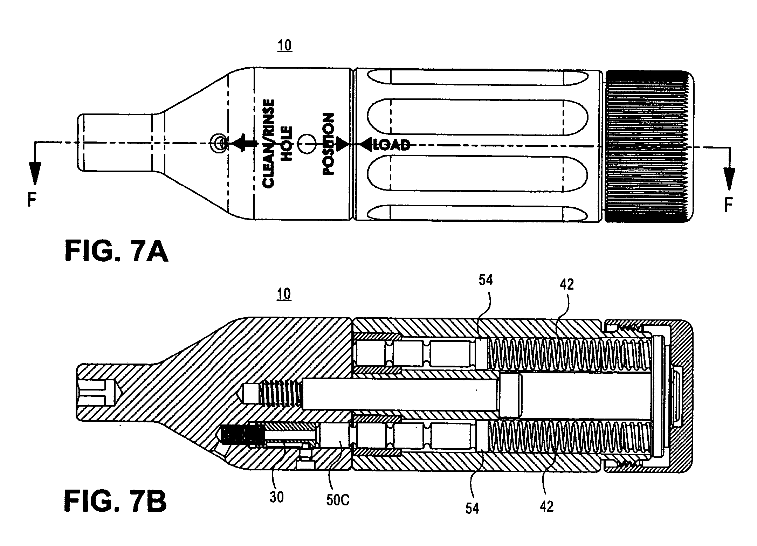

FIG. 7A is a top view of the torque limiting device 10 in a second loaded position. The handle 16 is now rotated to the next "Load" position.

FIG. 7B is a cross-sectional view F-F of the torque limiting device 10 in the second loaded position. As can be seen in the cross-sectional view, the first segment 50C of a second insert 40 advances into the shear component 22, pushing the ejector pin 30 until it stops. Note that the first segment 50C will not advance until it aligns with the hole 26A in the shear component 22.

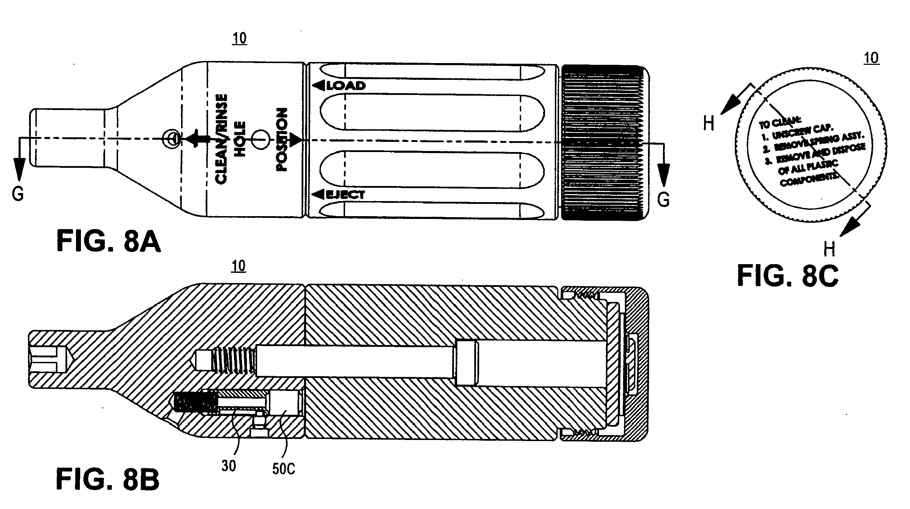

FIG. 8A is a top view of the torque limiting device 10 in a second shear position. When the second insert 40 shears at the pre-determined torque value, the handle 16 will be in an in-between position of "Load" and "Eject".

FIG. 8B is a cross-sectional view G-G of the torque limiting device 10 in the second shear position. In the above view the front segment 50C of the second insert 40 is shown sheared and retained inside the hole 26A of the shear component 22. Because there is only the one hole on the shear component 22 that is on the same bolt circle as the second insert 40, the second insert 40 will not advance to the next position.

FIG. 8C is an end view of the torque limiting device 10 in the eject position. As shown in FIG. 8C, the section line H-H is disposed at an angle to pass through the loading bores 26B.

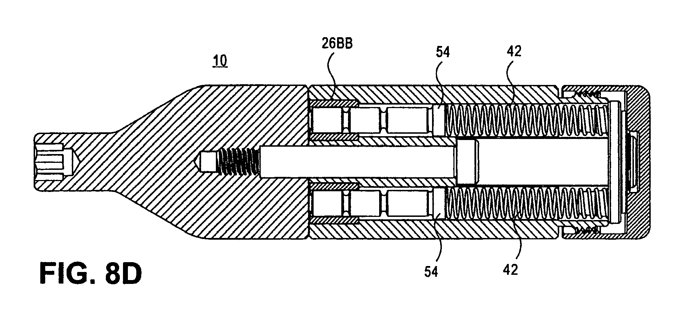

FIG. 8D is a cross-sectional view H-H of the torque limiting device 10 in the eject position. As shown in FIG. 8D, the cross-sectional view H-H transects the loading bore 26B and illustrates how both the first insert 40 and second insert 40 are essentially prevented from advancing while the shear component 22 is between the load eject positions.

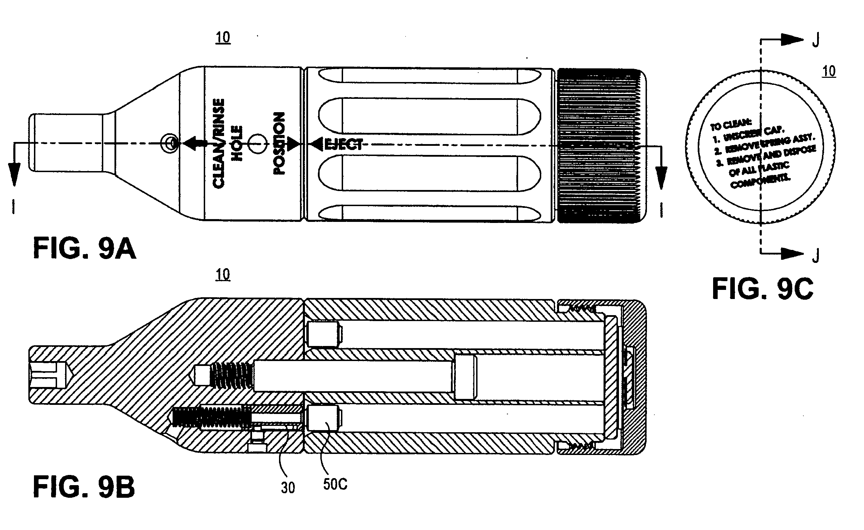

FIG. 9A is a top view of the torque limiting device 10 in a second eject position. In response to rotating the handle 16 the torque limiting device 10 advances to an "Eject" position.

FIG. 9B is a cross-sectional view I-I of the torque limiting device 10 in the second eject position. As shown in the transparent view above, the segment 50C of the second insert 40 that has been sheared off will now advance into ejection chamber of the handle 16. Because of the limit slot 34 in the ejector pin 30 it will not advance into the ejection chamber. Note that there are now two separate insert 40 (e.g., the first and second insert 40) segments in two separate ejection chambers.

FIG. 9C is an end view of the torque limiting device 10 in the second eject position. As shown in FIG. 9C, the section line J-J is disposed at an angle to pass through the loading bores 26B.

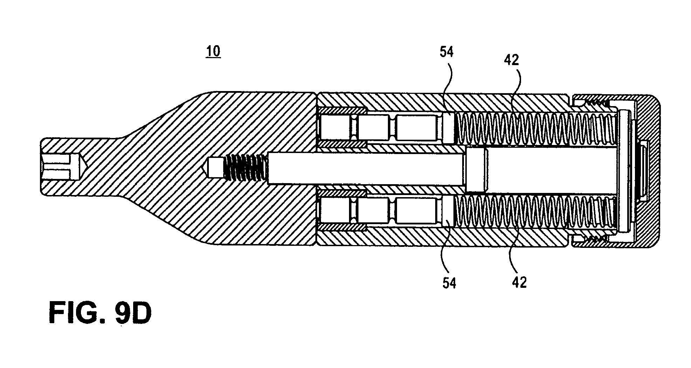

FIG. 9D is a cross-sectional view J-J of the torque limiting device 10 in the second eject position. As shown in FIG. 9D, the cross-sectional view J-J transects the loading bore 26B and illustrates how both the first insert 40 and second insert 40 are essentially prevented from advancing while the shear component 22 is in the eject position.

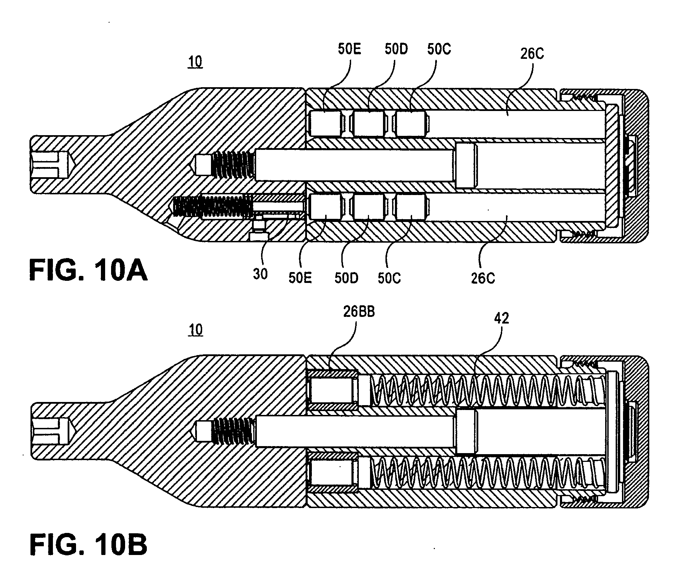

FIG. 10A is a cross-sectional view of the torque limiting device 10 in a final eject position. As shown in FIG. 10A, the segments 50C-50E are retained within the collecting bores 26C

FIG. 10B is a cross-sectional top view of the torque limiting device 10 in the final eject position. As shown in FIG. 10B, the flange 54 is configured to engage a lip or otherwise essentially prevent the insert 40 from progressing further than the final segment.

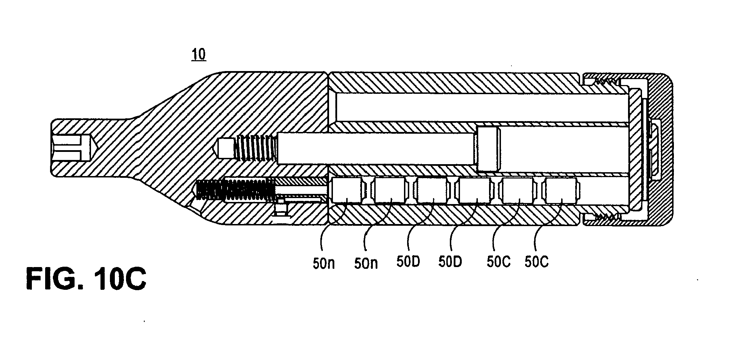

FIG. 10C is a cross-sectional top view of the torque limiting device 10 in the final eject position according to another embodiment. As you can see from the cross-sectional views above, the torque limiting device 10 according to this particular embodiment is capable of six torque-limiting uses. In other embodiments, the torque limiting device 10 and/or the insert 40 may be configured for more or fewer torque-limiting uses. Once the segments 50C-50n are all sheared off the flange 54 will prevent the insert 40 from advancing further. At this point the device 10 will only spin. The device 10 is also designed to hold all six segments 50A-50n.

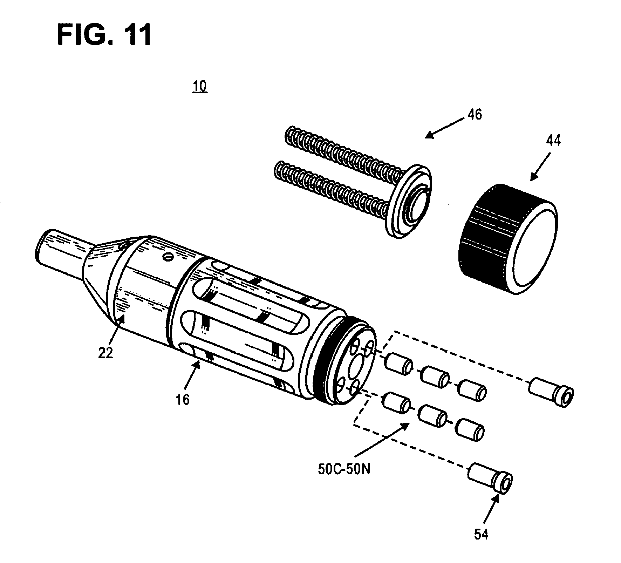

FIG. 11 is an exploded view illustrating removal of the used insert 40 from the torque limiting device 10 of FIG. 1.

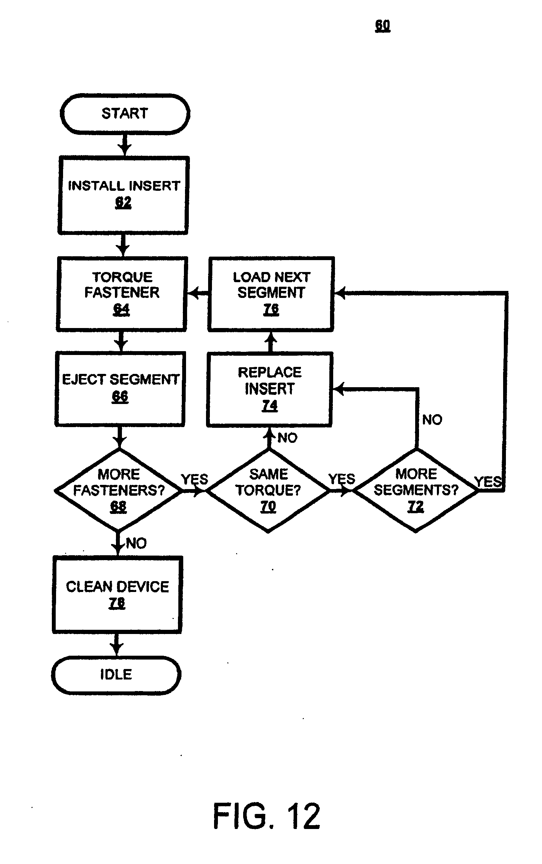

FIG. 12 is a flow diagram illustrating a method 60 of installing a fastener according to an embodiment of the invention. As shown in FIG. 12, the method 60 may be initiated at step 62 in response to installing a torque limiting insert such as the insert 40 in the torque limiting device 10. To initiate installation, loading sleeves or bores may be accessed. For example, the retaining cap 44 may be unscrewed or otherwise removed along with the spring 42 and spring assembly 46 to expose the loading bore 26B and the collecting bore 26C. Thereafter, the insert 40 may be positioned in the loading bore 26B and the torque limiting device reassembled. Of note, the insert 40 installed in the torque limiting device 10 may be selected based upon a predetermined torque limit for a particular fastener that is to be installed. For example, a fastener such as a bone screw may have a recommended installation torque of 4 Newton Meters (Nm). The insert 40 selected for installation may include a predetermined shear strength that corresponds to 4 Nm of torque.

At step 64, the fastener 12 may be installed in the substrate 14. For example, a bone screw may be screwed into the bone of a patient. More particularly, the fastener 12 may be coupled to the torque limiting device 10 by a bit and the handle 16 may be rotated until the insert 40 is sheared as shown in FIG. 5A and 5B.

At step 66, a severed segment may be ejected from the head 20. For example, as shown in FIGS. 6A and 6B, the shearing hole 26A may be aligned with the collecting bore 26C. In response, the segment 50C, for example, may be urged by the spring loaded ejector pin 30 and spring 32 into the collecting bore 26C.

At step 68, it may be determined if additional fasteners are to be installed. For example, if an ongoing operational procedure indicates addition fasteners may be utilized, it may be determined if the additional fasteners include essentially the same torque indication at step 70. If no additional fastener installation is indicated, the torque limiting device 10 maybe cleaned at step 78.

At step 70, it may be determined if essentially the same torque limit is indicated. For example, essentially the same torque limit may be indicated in situations utilizing the same type of bone screw being installed into similar boney tissue. If it is determined that essentially the same torque limit is indicated, it may be determined if additional segments 50C-50n remain attached to the insert 40. If it is determined that a different torque limit is indicated, the insert 40 may be exchanged at step 74.

At step 72, it may be determined if more segments remain. For example, if upon rotating the handle 16 relative to the head 20 one full rotation, no segment 50C-50n is loaded into the shearing hole 26A, then it may be determined that no segments remain and the insert 40 may be replaced at step 74. If segments 50C-50n do remain, a next segment may be loaded if not already done so at step 76.

At step 74, the insert 40 may be replaced. For example, the retaining cap 44 may be unscrewed or otherwise removed along with the spring 42 and spring assembly 46 to expose the loading bore 26B and the collecting bore 26C. As shown in FIG. 11, the insert 40 and any spent segments 50C-50n may be removed via the assistance of gravity by tilting the torque limiting 10. Thereafter, the insert 40 having a torque limit corresponding to a next fastener to be installed in the loading bore 26B as shown in FIG. 3C and the torque limiting device reassembled.

At step 76, a next segment 50C-50n may be loaded. For example, if a segment has not already been loaded, the head 20 may be rotated relative to the handle 16 until the load position is obtained as shown in FIGS. 7A and 7B. Following loading, a fastener may be installed at step 64.

At step 78, the torque limiting device 10 may be cleaned. For example, the torque limiting device 10 may be disassembled as shown in FIG. 11 and the torque limiting device 10 may be washed. Once emptied of inserts 40, the torque limiting device may be reassembled and/or sterilized. In a particular example, the torque limiting device 10 may be autoclaved.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. An apparatus comprising:
a head having a central rotational axis;
a handle that is rotatably fastened to the head, wherein the handle and head mate at an interface;
the head defining a head axial bore offset from the central rotational axis; and
the head defining a handle axial bore offset from the central rotational axis, the handle axial bore being shaped to receive a torque limiting insert, wherein in response to the head being at a first rotational position relative to the handle, the head axial bore and handle axial bore are disposed in cooperative alignment to allow the torque limiting insert to transect the interface and enter the head axial bore, wherein the head and the handle are rotationally coupled via the torque limiting insert, wherein the head and the handle are rotationally uncoupled in response to a shear force being applied across the interface that exceeds a shear limit for the torque limiting insert.

2. The apparatus according to claim 1, further comprising:
a second handle axial bore defined by the handle and offset from the central rotational axis, the second handle axial bore being configured to receive a sheared portion of the torque limiting insert, wherein in response to the head being at a second rotational position relative to the handle, the head axial bore and second handle axial bore are disposed in cooperative alignment to allow the sheared portion of the torque limiting insert to exit the head axial bore and enter the second handle axial bore.

3. The apparatus according to claim 2, further comprising:
a return spring biased to urge the sheared portion of the torque limiting insert towards the handle.

4. The apparatus according to claim 1, further comprising:
an annular groove disposed about the torque limiting insert.

5. The apparatus according to claim 1, further comprising:
a spring biased to urge the torque limiting insert towards the head.

6. The apparatus according to claim 1, further comprising:
a removable base assembly to access the first handle bore.

7. The apparatus according to claim 6, further comprising:
a textured surface on the removable base assembly to facilitate removal of the base assembly from the handle.

8. The apparatus according to claim 1, further comprising:
a clearing port to clear a sheared portion of the torque limiting insert from the head.

9. The apparatus according to claim 1, further comprising:
a gripping surface to facilitate securely grasping the handle.

10. An apparatus for applying a predetermined amount of torque to a fastener, the apparatus comprising:
a head means for transmitting torque to the fastener;
a handle means coupled to the head means for applying an amount of torque to the fastener; and
a torque limiting means interoperably disposed between the head means and the handle means to limit an applied torque from being transmitted from the handle means to the head means by shearing a torque limiting insert in response to the amount of torque being greater than the predetermined amount of torque, wherein the handle means is rotationally decoupled from the fastener in response to the torque limiting insert being sheared.

11. The apparatus according to claim 10, wherein the torque limiting insert spans an interface between the head means and the handle means, wherein shearing the torque limiting insert generates a detached segment.

12. The apparatus according to claim 11, further comprising:
means for urging the detached segment out of the head means and into a storage compartment of the handle means.

13. The apparatus according to claim 11, further comprising:
means for urging the torque limiting insert to span the interface between the head means and the handle means in response to a handle bore being aligned with a head bore.

14. The apparatus according to claim 10, further comprising:
means for modulating the predetermined amount of torque in response to replacing a first torque limiting insert with a second torque limiting insert.

15. A method of applying a predetermined amount of torque upon a fastener, the method comprising:
coupling a torque limiting device to the fastener, wherein the torque limiting device includes a torque limiting insert; and
applying an amount of torque upon the fastener by urging a handle of the torque limiting device to rotate, the torque limiting insert being subjected to a shear force in response to the amount of torque and the torque limiting insert shearing in response to the amount of torque being greater than the predetermined amount of torque, wherein the handle is rotationally decoupled from the fastener in response to the torque limiting insert being sheared.

16. The method according to claim 15, further comprising:
spanning an interface between a head of the torque limiting device and the handle with a portion of the torque limiting insert, wherein shearing the torque limiting insert generates a detached segment.

17. The method according to claim 16, further comprising:
urging the detached segment out of the head and into a storage compartment of the handle.

18. The method according to claim 16, further comprising:
urging the torque limiting insert to span the interface between the head of the torque limiting device and the handle in response to a handle bore being aligned with a head bore.

19. The method according to claim 15, further comprising:
modulating the predetermined amount of torque by replacing the torque limiting insert with a second torque limiting insert.
